(19) Europäisches Patentamt
European Patent Office
Office européen des brevets

(11) EP 1 394 245 B1

(12) **EUROPEAN PATENT SPECIFICATION**

(45) Date of publication and mention of the grant of the patent:
**09.06.2010 Bulletin 2010/23**

(21) Application number: **02733412.7**

(22) Date of filing: **07.06.2002**

(51) Int Cl.:
*C11D 17/08* (2006.01) *C11D 7/22* (2006.01)
*A61L 2/16* (2006.01) *A61L 2/18* (2006.01)
*C11D 7/50* (2006.01) *C11D 7/10* (2006.01)
*C11D 3/00* (2006.01) *A01N 59/08* (2006.01)

(86) International application number:
**PCT/JP2002/005689**

(87) International publication number:
**WO 2002/100995 (19.12.2002 Gazette 2002/51)**

(54) **METHOD FOR REMOVAL OF ALLERGENS**

METHODE ZUR ENTFERNUNG VON ALLERGENEN

METHODE POUR L'ELIMINATION D'ALLERGENES

(84) Designated Contracting States:
**DE FR GB**

(30) Priority: **08.06.2001 JP 2001173477**
**13.07.2001 JP 2001213373**
**31.07.2001 JP 2001231689**
**12.10.2001 JP 2001315631**

(43) Date of publication of application:
**03.03.2004 Bulletin 2004/10**

(73) Proprietor: **KAO CORPORATION**
**Tokyo 103-8210 (JP)**

(72) Inventors:
- **NAGAI, Satoshi, c/o Kao Corporation Wakayama-shi, Wakayama 640-8580 (JP)**
- **TAKANO, Katsuyuki, c/o Kao Corporation Wakayama-shi, Wakayama 640-8580 (JP)**
- **SUZUKI, Masahiro, c/o Kao Corporation Wakayama-shi, Wakayama 640-8580 (JP)**
- **BAN, Takeshi, c/o Kao Corporation Wakayama-shi, Wakayama 640-8580 (JP)**
- **YOKOSUKA, Michio, c/o Kao Corporation Wakayama-shi, Wakayama 640-8580 (JP)**

(74) Representative: **HOFFMANN EITLE**
**Patent- und Rechtsanwälte**
**Arabellastrasse 4**
**81925 München (DE)**

(56) References cited:
**EP-A- 0 168 243** **EP-A- 0 259 499**
**EP-A- 0 424 845** **WO-A-98/50518**
**WO-A1-95/34630** **DE-A1- 3 633 242**
**GB-A- 2 058 820** **JP-A- 2000 063 207**

- **DATABASE WPI Section Ch, Week 199851 Derwent Publications Ltd., London, GB; Class D16, AN 1998-608189 XP002325908 & RU 2 112 029 C1 (MOSC KRISTALL WKS STOCK CO) 27 May 1998 (1998-05-27)**
- **DATABASE WPI Section Ch, Week 199444 Derwent Publications Ltd., London, GB; Class B05, AN 1994-354630 XP002325909 & JP 06 279273 A (EARTH SEIYAKU KK) 4 October 1994 (1994-10-04)**
- **DATABASE WPI Section Ch, Week 199817 Derwent Publications Ltd., London, GB; Class D16, AN 1998-189851 XP002332889 & JP 10 046467 A (SHINWA KOGYO KK) 17 February 1998 (1998-02-17)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 394 245 B1

- **DATABASE WPI Section Ch, Week 200135 Derwent Publications Ltd., London, GB; Class A25, AN 2001-331552 XP002332890 & JP 2001 046795 A (SANKO KAGAKU KK) 20 February 2001 (2001-02-20)**
- **DATABASE WPI Section Ch, Week 199038 Derwent Publications Ltd., London, GB; Class A82, AN 1990-287208 XP002332891 & JP 02 202978 A (KAO CORP) 13 August 1990 (1990-08-13)**
- **DATABASE WPI Section Ch, Week 199824 Derwent Publications Ltd., London, GB; Class E19, AN 1998-267370 XP002332892 & JP 10 088113 A (LION CORP) 7 April 1998 (1998-04-07)**
- **KINGLAKE V.: 'Formulating liquid detergents for domestic household use' SOAP, PERFUMERY & COSMETICS vol. 54, no. 3, 1981, page 151, 153, 155, 159, XP002956601**
- **'Carpet-yo senzai no hikaku test kekka.' SHOWA 62 NEN, NATIONAL CONSUMER AFFAIRS CENTER OF JAPAN December 1987, pages 1 - 23, XP002966376**
- **KUWAMURA H. ET AL: 'Interior sen'i seihin no senjo' SEN'I SEIHIN SHOHI KAGAKU vol. 23, no. 7, 1982, pages 14 - 19, XP002966377**


Remarks:

The file contains technical information submitted after the application was filed and not included in this specification

## Description

Technical field of the Invention

The present invention relates to a method for removing effectively allergens which are not easily removed by ordinary cleaning, the method making it possible effectively to remove allergens, which are substances which become antigens of allergies, such as dead mite or dung of mites, spores of fungi, and pollen.

Prior art

It appears that mites, chironomids, cockroaches, dust and dirt resulting from dead bodies or dung thereof, fragments of body hairs of pets, pollen, and spores of fungi, which substance are antigens, induce allergic diseases. Effects of the so-called allergens upon human bodies are becoming social problems. Particularly about mites, the propagation thereof is promoted in exchange for comfortableness of the indoor environment, and the presence of causative agents of allergic diseases such as asthma, atopic dermatitis, allergic rhinitis and so on becomes obvious. Particularly mites such as Dermatophagoides pteronyssinus (Trouessart), Tyrophagus putrescentiae (Schrank), Dermatophagoides farinae Hughes inhabit and propagate in floor coverings such as a tatami mat or carpet, bed lines such as a coverlet, and so on. Not only people having an allergic disease but also ordinary families are extremely interested in extermination of such mites. A great number of techniques are suggested about anti-mite agents or miticides. For example, JP-A No. 9-157116 describes an anti-mite agent comprising phenylisothiocyanate as an anti-mite component, and further comprising a perfume selected from sesquiterpene compounds, aliphatic compounds and aromatic compounds as an agent for relieving the irritating odor of phenylisothiocyanate. However, it is known that dead mites or dung of mites also become allergens. Thus, investigations have been made to make these allergens harmless.

JP-A No. 6-279273 discloses a method of removing an allergen, using a specific compound such as gallic acid or a lower alcohol ester thereof. JP-A No. 61-44821 discloses an agent for removing allergens, comprising tannic acid and a specific solvent. JP-A No. 2000-63207 discloses the fact that a specific solvent is useful for making an allergen harmless.

However, regarding these allergen-removing methods or agents in the prior art, the following fact is called "removal": the fact that a protein is denatured to lower the activity of bonding to an IgE antibody, which is a factor of the pathogenesis of an allergy, thereby making the allergen harmless. Thus, it would be proper that the agent is called an allergen-denaturing agent. Moreover, the allergen or a precursor thereof still remains after the treatment with the agent. Therefore, the effect of the allergen is insufficiently excluded. When people are repeatedly exposed to an allergen denatured by these techniques, the denatured allergen itself becomes a new allergen substance, which may cause the pathogenesis of an allergy.

Incidentally, about allergens resulting from organisms, such as mites, dung of mites, pollen or spores of fungi, it is known that careful cleaning thereof with a cleaning tool such as a vacuum cleaner is useful. However, in floor coverings such as a tatami mat and carpet, and bed lines such as a blanket and a coverlet, allergen substances are present even in deep parts thereof. Thus, the allergen substances cannot be easily removed. As a method of removing mite allergens effectively from the indoor environment, the following methods are recommended: a method of vacuuming with a vacuum cleaner on a floor for 20 seconds or more per $m^2$, or a method of cleaning bed lines carefully with a vacuum cleaner one time per week. It is however very difficult to carry out such a method routinely. For this reason, there is desired a method of removing an allergen effectively by a simple cleaning operation.

A method of cleaning carpet is known by way of spraying a powder impregnated with a cleaning liquid such as a cleaning agent for carpet in which mites are living, rubbing the powder and the carpet together with a brush and removing the powder with a vacuum cleaner. Further as other cleaning agents for carpet, JP53-130704 discloses a liquid composition for shampooing carpet, impregnated with water to include a solid in an amount of 10 to 40%, containing a lithium salt of polyphosphoric acid including a lithium salt of sulfates of a mixed alcohol obtained by mixing 80 to 95 parts of lauryl alcohol with 5 to 20 parts of decyl alcohol and 70 to 85% of $P_2O_5$ as a builder, in addition referring to jetting out the carpet shampoo diluted with water, extending it over the carpet's surface, brushing the carpet, drying it and removing the powder residue of the liquid by vacuum. GB-A 1 3 4 3 3 1 2, an equivalent to JP-A 4 6 - 2 9 3 4, describes a cleaning composition for carpet including a water-soluble organic surfactant, a swelling fatty alcohol and a water-insoluble silicic acid and a process for making foam of the composition on carpet and treating it, after the form has disappeared and dried, with a vacuum cleaner. A cleaning product is commercially available in Japan, in which a carpet is cleaned by spraying a liquid industrial carpet cleaner, polishing the carpet with a polisher and vacuuming the dried powder. It is known to clean carpets by applying powder or liquid and vacuum dried powder with a vacuum cleaner as described above. The known carpet cleaner, however, has the purpose to remove soils from the carpets, and contains principally a surfactant or a powdery absorber, and does not have the purpose to remove allergens.

Disclosure of the Invention

Accordingly, a purpose of the present invention is to provide an allergen-removing method which makes it possible to remove allergen substances effectively by a simple cleaning operation and further does not change the feel of the object to be treated.

The present invention provides an allergen-removing method on the basis of a completely new system, which has never been known so far.

The present invention relates to a method for removing allergens, with an allergen-removing agent, which comprises bringing a liquid composition which generates a solid by removing liquid components thereof into contact with an object to be treated in which an allergen is present and drying the contacted place to form a precipitated solid and removing, from the object, the allergen together with the precipitated solid,

the allergen-removing agent comprising the following component (a), component (b) and component (c2): wherein

component (a) at least one compound selected from the group consisting of ethanol, 1-propanol and 2-propanol,

component (b): water, and

component (c2): a solid source substance which is soluble in at least one of component (a) and component (b), and generates a solid by evaporation of liquid components of the allergen-removing agent,

being a combination of an inorganic cation with an inorganic anion, the inorganic cation being at least one selected from the group of alkali metal ions and alkaline earth metal ions, the inorganic anion being at least one selected from the group of a sulfate ion, a carbonate ion and a phosphate ion,

the allergen-removing agent comprising 1-70% by mass of component (a), 30-98.999% by mass of component (b) and 0.001-5% by mass of component (c2).

Usually, the allergen in the present invention broadly represents causative substances of allergies. In the present invention, the allergen represents particularly mites, chironomids, cockroaches, dead bodies thereof, dust and dirt resulting therefrom, fragments of body hairs of pets, dry matters of proteins in saliva of pets, pollen, and spores of fungi. The allergen-removing agent to be used according to the present invention is effective for removing allergens having a size of 100 μm or less, which are not easily removed by cleaning.

Detailed description of the Invention

<Component (a)>

Component (a) of the present invention is at least one of ethanol, 1-propanol and 2-propanol. By using component (a) together with water, the drying of an object which is treated with the allergen-removing agent is promoted so that the generation of a solid resulting from component (c) is promoted.

<Component (b) >

Component (b) of the present invention is water, is the balance of the agent comprising component (a), component (c) and other components, and is also a solvent for a part of component (c). As to water, water containing a little amount of metal ions (which may be component (c)) may be used. It is however preferred to use ion-exchanged water from the viewpoint of preservation stability.

<Component (c)>

Component (c) is the most important component in the present invention. Component (c) has a nature of being dissolved in component (a) and/or component (b) and, after drying of the solution, generating component (c) itself or a solid resulting from component (c). Component (c) is a solid source substance which is soluble and present at least in component (a) and/or component (b), and generates a solid by evaporating liquid components of the allergen-removing agent. The evaporation of the liquid components does not mean that all the liquid components are evaporated, but a sufficient amount of the liquid components are evaporated so as to generate a solid. That is, the liquid components in the allergen-removing agent of the present invention are made mainly of component (a) and component (b). However, in the case that a small amount of liquid components having a low volatility, such as a perfume material which will be described later, is contained therein, these may not necessarily be evaporated. After the allergen-removing agent of the present invention is sprayed or applied onto an object to be treated in which an allergen is present, the incorporation of component (c) having a nature as described above causes the generation of a solid by drying the treated portion. It appears that the generated solid carries the allergen or the allergen is easily removed from the surface of the object to be treated.

In the present invention, component (c) comprises component (c2), but may optionally contain component (c1):

component (c1): an organic compound which is in a solid state at 1013.25 hPa and 25°C, and
component (c2): a certain combination of an inorganic cation with an inorganic anion.
Component (c1) of these components is preferably at least one selected from the following components (c1-1), (c1-2) and (c1-3).
component (c1-1) : an organic compound having a melting point of 25°C or more [provided that components (c1-2) and (c1-3) are excluded],
component (c1-2): a surfactant which is in a solid state with a water content of 5% or less by mass, and
component (c1-3): citric acid, succinic acid, tartaric acid, malic acid, fumaric acid, malonic acid, maleic acid, adipic acid, lactic acid, phthalic acid, terephthalic acid, asparagic acid, azelaic acid, glutamic acid, glutaric acid, oxalic acid, glycine, and alkali metal salts or alkali earth metal salts thereof.
Component (cl-1) preferably has a melting point of 35°C or more when a hot season such as summer is considered. Component (c1-1) is preferably a component having a low hygroscopic property so that the treated allergen may be easily removed from the surface of the obj ect to be treated. From the viewpoint of the hygroscopic property, it is preferable that the compound has a hygroscopic degree, determined by the following hygroscopic property test, of from 0 to 2, more preferably from 0 to 1, particularly preferably from 0 to 0.5.

(Hygroscopic property test)

Component (c1-1) is pulverized and then the powder is classified into a size of 500 to 1000 $\mu$m with sieves. One gram (Wa) of the classified components is put into a columnar petri dish made of glass and having a diameter of 7 cm and a depth of 1.5 cm. The mass of the whole is measured (Wb). Next, the dish is allowed to stand still in a thermostatic chamber of 80% humidity and 20°C temperature for 24 hours. Thereafter, the whole mass is measured (Wc), and then the hygroscopic degree is calculated from the following equation:

$$\text{hygroscopic degree} = (Wc - Wb)/Wa$$

Component (c1-1) is preferably a component having poor solubility in water. Specifically, the solubility in water at 20°C is preferably 0.02 g/100 g or less, and more preferably 0.01 g/100 g or less. Component (c1-1) is preferably in a state that it is soluble in the allergen-removing agent. Therefore, the solubility thereof in component (a) is preferably 0.05 g/100 g or more, and more preferably 0.1 g/100 g or more. The solubility herein can be obtained by a method described in item "Solubility test" on page 399 in "Kagaku Daiziten (Chemistry Great Dictionary) 9 (Kyoritsu Shuppan Co.)".
Specific preferred examples of component (c1-1) are compounds selected from the following components (c1-1-1), (c1-1-2) and (c1-1-3):

component (c1-1-1): an alicyclic compound having a melting point of 40 to 250°C, preferably 60 to 210°C, and having 10 to 25 carbon atoms, preferably 10 to 20 carbon atoms.
component (c1-1-2): an aliphatic compound having a melting point of 35°C or more, preferably 35 to 200°C, and having 8 to 36 carbon atoms, preferably 12 to 20 carbon atoms, and
component (c1-1-3): an aromatic compound having a melting point of 40 to 200°C and having 7 to 24 carbon atoms, preferably 7 to 20 carbon atoms.

Specific examples of the compound of component (c1-1-1) include camphene, *1*-menthol, borneol, cedrol, t-butylcyclohexanol, camphor, p-t-butylcyclohexanone, maltol, cyclopentadecanone, hinokitiol, caliofilen oxide, and 1,5-Dimethylbicyclo[3.2.1]octan-8-one-oxime ("Buccoxime", Doragoco GmbH).
Specific examples of the compound of component (c1-1-2) include lauric acid, myristic acid, palmitic acid, stearic acid, myristyl alcohol, palmityl alcohol, stearyl alcohol, and n-tetradecylaldehyde.
Specific examples of the compound of component (c1-1-3) include dimethylphenyl carbitol, phenylglycol, vanillin, ethylvanilin, benzophenone, methyl naphthyl ketone, coumalin, musk xylene, musk ketone, musk ambrette, musk tybetene, 1,1,3,3,5-Pentamethyl-4,6-dinitroindane(Musk moskene, "Muskmoskene", Givaudan SA), 4-Acethyl-6-t-butyl-1,1-dimethylindane("Celestolide", International Flavors & Fragrances Inc.), 7-Acethyl-1,1,4,4-tetramethyl-6-ethyl-1,2,3,4-tetrahydronaphthalene("Versalide",Givaudan SA), 6-acetyl-1,1,2,4,4,7-hexamethyl-tetraline ("Tonalide", PFW Aroma Chemical), dimethylhydroquinone, thymol, trans-benzylisoeugenol, β-naphtholmethyl ether, benzoic acid, cinnamic acid, phenylacetic acid, hydrocinnamic acid, acetic acid isoeugenol, cinnamyl cinnamate, phenylethyl salicylate, methyl anisate, indole, skatole, rosephenone, methyl athralate, rasbery ketone, heliotropylacetone, 3-methyl-4-isopropylphenol, and p-chloro-m-xylenol. These compounds of (c1-1-3) have a solubility in water of 0.02 g/100 g or less at 20°C, and a solubility in component (a) of 0.05 g/100 g or more at 20°C.

In the present invention, component(c-1-1) is especially preferably cedorol, *1*-mentol, myristyl alcohol, palmityl alcohol, stearyl alcohol, camphor or thimol.

From component (c1-1) of the present invention, a surfactant such as component (c1-2), which will be described later, and an organic acid of component (c1-3) or a salt thereof, which will be described later, are excluded. The surfactant of the present invention is a compound having a critical micelle concentration (c. m. c.), an HLB, which is obtained by the method described on page 319 of Surfactant Handbook (published by Sangyo Tosho Co., Ltd. in 1960), of 6 or more, or a nature of the two. Compounds not having these properties and having a melting point of 25°C or more are handled as component (c1-1).

Component (c1-2) is a surfactant which is in a solid state at 25°C, preferably 35°C, when the water content therein is 5% or less by mass. Component (c1-2) used in the present invention is preferably a compound having a hygroscopic degree of 0 to 2, preferably 0 to 1 and more preferably 0 to 0.5, which is according to the above-mentioned hygroscopic property test.

Component (c1-2) is preferably a nonionic surfactant having an alkyl group having 8 to 20 carbon atoms or an anionic surfactant having a saturated alkyl group having 14 to 24, preferably 14 to 20 carbon atoms. When the carbon number is less than 12, it easily remains because the surfactant gets viscous. When the carbon number is more than 20, a branched alkyl is preferably used. It is particularly preferably selected from the following components (cl-2-1) to (c1-2-3) :

component (c1-2-1): an anionic surfactant having a saturated alkyl group having 14 to 20 carbon atoms, and having a sulfuric ester group and/or a sulfonic acid group,
component (c1-2-2) : a polyoxyalkylene alkyl (alkenyl) ether wherein 20 to 150 mols of alkylene oxide, preferably ethylene oxide (hereinafter referred to as EO), are added to a saturated aliphatic alcohol having 8 to 20 carbon atoms, and
component (c1-2-3): a saturated aliphatic acid soap having 14 to 18 carbon atoms.

The most preferable example of component (c1-2) is an alkyl sulfate ester having 14 to 20 carbon atoms. The counter ion is preferably sodium.

Preferred examples of component (c1-3) of the present invention include citric acid, succinic acid, tartaric acid, malic acid, fumaric acid, malonic acid, maleic acid, adipic acid, phthalic acid, terephthalic acid, and salts thereof (preferably, sodium salts and/or potassium salts). Phthalic acid and salts thereof are most preferred from the viewpoint of allergen-removing effect.

It is defined that all surfactants of component (c1-2) of the present invention are in a solid state at 25°C, preferably 35°C, when their water content is 5% or less by mass. When the water content is over 5% by mass, the state of the surfactant can be checked by adding ethanol or isopropanol thereto, subjecting the solution to azeotropic dehydration with an evaporator under reduced pressure to make the water content 5% or less by weight, putting the resultant into a columnar glass petri dish having a diameter of 7 cm and a depth of 1.5 cm, allowing the dish to stand still in a thermostat of 25°C temperature, preferably 35°C temperature, for 24 hours, and observing the state of component (c1-2) with the eye. Alternatively, it can also be checked by the presence of an endothermic peak based on melting at 25°C or more, preferably 35°C or more in DSC (differential scanning calorimetry). The water content can be determined by the Karl Fischer technique (JIS K 33625).

Component (c2) is a combination of an inorganic cation with an inorganic anion, wherein the inorganic cation is an alkali metal or alkaline earth metal and the inorganic anion is at least one selected from a sulfate ion, a carbonate ion and a phosphate ion. Component (c2) is particularly preferably a combination of a cation (c2-c) selected from a potassium ion, a sodium ion and a magnesium ion with an anion as defined above (c2-a). However, it is difficult that a combination of only potassium and carbonate ions is solidified.

In the present invention, therefore, it is preferred to blend an inorganic salt which is composed of a cation selected from a potassium ion, a sodium ion and a magnesium ion and an anion selected from a sulfate ion and a carbonate ion and which has a pH of 3 to 9 at 25°C in the case of an aqueous solution containing 1% by mass of the salt. Among these inorganic salts, particularly preferred are compounds having a solubility in water of 10 to 50%, preferably 15 to 40%, by mass at 25°C. The solubility of this inorganic salt is a value representing a mass of the inorganic compound contained in 100 g of a saturated aqueous solution in % by mass, and is described in II-166 to II-176 of Kagaku Binran Kiso-Hen (Chemistry Handbook Basic Version) II, revised 3rd edition, edited by the Chemical Society of Japan, and published by Maruzen Co., Ltd. Furthermore, preferred inorganic salts in the present invention are compounds having a hygroscopic degree of 0 to 2, preferably 0 to 1 and more preferably 0 to 0.5, which is measured according to the following hygroscopic property test.

(Hygroscopic property test)

Ten grams of an inorganic salt as a supply source of component (c) is dissolved in 100 mL of water, and the solution is put into a stainless steel vat having a length of 20 cm, a width of 16 cm and a depth of 3 cm. With a vacuum dryer, water is removed (at the time of drying, the temperature is 30°C, the vacuum degree is 200 mmHg, and drying time is 1 week). The precipitated crystal is pulverized and classified into a size of 500 to 1000 μm with sieves. One gram (W1) of the classified components is put into a columnar petri dish made of glass and having a diameter of 7 cm and a depth of 1.5 cm. The mass of the whole is measured (W2). Next, the dish is allowed to stand still in a thermostatic chamber of 80% humidity and 20°C temperature for 24 hours. Thereafter, the whole mass is measured (W3), and then the hygroscopic degree is calculated from the following equation:

$$\text{hygroscopic degree} = (W3 - W2)/W1$$

More specific preferred examples of the inorganic salt include sodium sulfate, magnesium sulfate, sodium carbonate, and sodium hydrogencarbonate. One or more selected from sodium sulfate, magnesium sulfate and sodium carbonate are preferred.

Component (c) of the present invention may be a mixture of components (c1) and (c2). In the case that component (c1-2) is an ionic surfactant or component (c1-3) is an organic acid salt, a counter ion thereof may be considered as part of component (c2). It is sufficient that the mixture is basically such a combination that causes precipitation of a solid from the allergen-removing agent containing component (c).

The organic compound included in scope by (c1), especially the surfactant of (c1-2), however, should be preferably used in a moderate amount because it happens to remain on the surface of the object.

<Component (d) >

Component (d) of the present invention is an organic compound other than component (a) and component (c), and is, for example, a highly polymerized compound such as an organic compound other than component (c1-1) (from which any surfactant is excluded), a surfactant other than component (c1-2), a polymer such as a polycarboxylic acid. Examples thereof include a perfume component, a fluorescent bleaching agent, an antibacterial agent, an antifungal agent, a sterilizer, an organic compound which is in a liquid state at 25°C, such as a surfactant or a glycol type solvent which is in a liquid state below 25°C, and a silicon oil which is in a liquid form at 25°C. Attention should be paid to the kind of component (d), and the amount thereof since it has effects on the precipitation of a solid from the allergen-removing agent.

In the present invention, a perfume component other than component (c1-1) [hereinafter referred to as component (d1)] is preferably selected as component (d). As component (d1), a component having mite-killing effect, mite-killing effect or the like is particularly preferred.

As component (d1), there may be used a component described in "Chemistry of Perfumes" (Industrial Chemistry Series written by Ryoichi Akaboshi, edited by the Chemical Society of Japan and published on September 16, 1983), "Chemistry and Commodity-Knowledge of Synthetic Perfumes" (written by Genichi Fuji, and published by Kagaku Kogyo Nippoh Co. on March 6, 1996) and "Practical Knowledge of Perfumes and Perfume-Preparation" (written by Motoki Nakajima, and published by Sungyo Tosyo Co., Ltd. on June 21, 1995). In order to improve the allergen-removing effect, it is preferred in the present invention to use at least one selected from components described in "Inducing agent, Repellents and Pheromone" in Chapter 2.6.4 and "Insecticides" in Chapter 2.6.5 in "Well-known and Conventional Techniques (Perfumes) Part 1, General Perfumes", and Japanese Patent Application No. 2001-94696.

Specifically, one or more selected from the following is particularly preferred: α-pinene, β-pinene, cis-3-hexenyl benzoate, hexyl benzoate, benzyl benzoate, eugenol, caryophylene, carbon, geraniol, cis-3-hexenyl salicylate, hexyl salicylate, santalol, citral, citronellal, citronellol, phenylethyl alcohol, hexylcinnamic aldehyde, benzyl alcohol, menthone, linalool, jasmone, dihydrojasmone, methyl jasmonate, methyl dihydrojasmonate, farnesol, nerolidol, phenoxyethanol, cineol, linalyl formate, cinnamic aldehyde, isophytol, phytol, cedryl methyl ether, β-damascone, α-damascone, β-ionone, α-ionone, tetrahydrolinalool, Lilarl (made by IFF Co.), benzaldehyde, n-amyl benzoate, isoamyl benzoate, heptyl benzoate, phenylethyl benzoate, n-butyl salicylate, isobutyl salicylate, n-amyl salicylate, isoamyl salicylate, methyl salicylate, benzyl salicylate, benzyl n-butyrate, benzyl isobutyrate, benzyl n-valerate, benzyl isovalerate, benzyl propionate, benzyl hexanoate, benzyl octanoate, and phenylpropyl alcohol. Most preferred are one or more selected from jasmone, dihydrojasmone, methyl jasmonate, methyl dihydrojasmonate, farnesol and nerolidol. In the present invention, such a perfume component can be used alone. A perfume composition composed of two or more of such components may be used.

In the present invention, it is also preferred to use, as component (d1), a natural essential oil extracted from

plants. Examples of the natural essential oil include oils of perpermint, almond, calamus, peppermint, spearmint, cinnamon, allspice, clove, thyme, rosemary, lemongrass, lemon, citron, lime, grape fruit, mandarin, ajowan, bergamot, laurel, star aniseed, majoram, mace, rosewood, palmarosa, lemonbelbana, lemonbaum, lavender, rose, orange, Japanese quassia, cassia, garlic, cajuput, perilla, cinnamon, camphor, cedarwood, patchouli, hinoki, white-cedar leaf, rice, pennyroyal, liceacubeba, eucalyptus, tea tree, ylang-ylang, vetiver, canaga, citronella, nutmeg, pepper, sandalwood, ginger, anise, mace and fennel.

Of these, preferred are one or more selected from ylang-ylang, orange, clove, sandalwood, spearmint, cedar wood, thyme, tea tree, Japanese mint, hinoki, white-cedar leaf, peppermint, pennylroyal, eucalyptus, lavender, lemongrass, lemon, rosemary, camphor, and ajowan oils.

These essential oils include compounds having a melting point of 25°C or more. In the present invention, however, compounds having a melting point of 25°C or more, among natural essential oils, are handled as component (c1-1).

From the viewpoint of the durability of the allergen-removing effect, it is preferred to use, as component (d1), one or more compounds selected from (d1-1) jasmoneoid, chained sesquiterpene alcohol, and chained diterpene alcohol. As component (d1-1), a compound having a boiling point of 230°C or more, preferably of 230 to 400°C, at 1013.25hPa is good from the viewpoint of durability of the allergen-removing effect. As the jasmomoid, jasmone, dihydrojasmone, lower-alkyl esters of jasmoneic acid (the carbon number of the alkyl group: 1-5), and lower- alkyl esters of dihydrojasmonic acid (the carbon number of the alkyl group: 1-5) are preferred. As the chained sesquiterepene alcohol, farnesol and nerolidol are preferred. As the chained diterpene alcohol, phytol, isophytol, geranylgeraniol and geranyllinalool are preferred. The most preferred components (d1-1) of the present invention are jasmone, dihydrojasmone, methyl jasmonate, ethyl jasmonate, methyl dihydrojasmonate, ethyl dihydrojasmonate, farnesol, nerolidol, and phytol. <Other additives>

Other components may be blended with the allergen-removing agent so far as the generation of a solid based on component (c) is not obstructed. Examples of the other components include a surfactant which does not come under component (c), a hydrotropic agent, a viscosity-adjuster, an antibacterial agent, an antifungal agent, and a pH adjuster. These components can also be placed as component (d). In order to adjust the pH, an alkali metal hydroxide, sulfuric acid, acetic acid or the like may be contained. Ions thereof are calculated as component (c2).

The surfactant which does not come under component (c) is preferably a nonionic surfactant, an anionic surfactant or a cationic surfactant particularly for the purpose of giving a transparent appearance to the composition and/or from the viewpoint of storage stability.

The nonionic surfactant is preferably a compound of the following general formula (1) and/or a compound of general formula (2):

$$R^{11}\text{-O-}(R^{12}O)_a\text{-H} \qquad (1)$$

wherein $R^{11}$ is an alkyl group or an alkenyl group having 8 to 18 carbon atoms, preferably 10 to 16 carbon atoms, $R^{12}$ is an alkylene group having 2 or 3 carbon atoms and is preferably an ethylene group, a is a number of 3 or more and less than 20, preferably 4 or more and 15 or less, and particularly preferably 5 or more and 10 or less, as an average addition mol number; and

$$R^{23}\text{-}(OR^{14})_bG_c\ x \qquad (1)$$

wherein $R^{13}$ is a linear alkyl group having 8 to 16 carbon atoms, preferably 10 to 16 carbon atoms and particularly preferably 10 to 14, $R^{14}$ is an alkylene group having 2 to 4 carbon atoms and is preferably an ethylene group or a propylene group, particularly an ethylene group, G is a residue originating from a reducing sugar, b is a number of average value 0 to 6, and c is a number of average value 1 to 10, preferably 1 to 5 and particularly preferably 1 to 2.

A particularly preferred compound among the compounds of general formula (1) may be a compound of the following general formula (1-1) or a compound of general formula (1-2) :

$$R^{15}\text{-O(EO)}_d\text{-H} \qquad (1\text{-}1)$$

wherein $R^{15}$ is a primary linear alkyl group, a branched alkyl or a secondary alkyl group having 10 to 18 carbon atoms, preferably 10 to 16 carbon atoms, EO is ethylene oxide, and d is 3 or more and less than 20 as an average addition mol number; and

$$R^{16}\text{-O[(EO)}_e\text{/(PO)}_f\text{]-H} \qquad (1\text{-}2)$$

wherein $R^{16}$ is a primary alkyl group having 10 to 18 carbon atoms, preferably 10 to 16 carbon atoms, EO is ethyleneoxide,

PO is propyleneoxide, e is an average addition mol number of 3 to 15, f is an average addition mol number of 1 to 5, the total of e and f is less than 20, and EO and PO may be a random adduct, a block adduct wherein PO is added after the addition of EO, or a block adduct wherein EO and PO are added in a manner reverse to the above.

In the compound of general formula (2), G is a residue originating from a reducing sugar. The reducing sugar as a raw material may be either aldose or ketose. Examples thereof include triose, tetrose, pentose, and hexose, which have 3 to 6 carbon atoms. Specific examples of aldose include apiose, arabinose, galactose, glucose, lyxose, mannose, agarose, aldose, idose, talose and xylose. An example of ketose is fructose. In the present invention, among these sugars, aldopentose or aldhexose, which has 5 or 6 carbon atoms, is preferred, and glucose is most preferred.

The anionic surfactant is preferably a surfactant selected from alkyl or alkenyl benzene sulfonates having an alkyl or alkenyl group having 10 to 18 carbon atoms.

As the alkyl or alkenyl benzene sulfonate, any surfactant selected from surfactants which are generally circulated in the market of surfactants for detergents can be used if the alkyl chain of the surfactant has 10 to 18 carbon atoms. For example, there may be used Neopelex F 25 made by Kao Corp., Dobs 102 made by Shell Co., or the like. It can be industrially obtained by sulfonating an alkylbenzene, which is widely circulated as raw material for detergents, with an oxidizer such as chlorosulfonic acid or sulfuric acid gas. The alkyl group preferably has 10 to 14 carbon atoms.

The salt is preferably a sodium salt, potassium salt, magnesium salt, calcium salt, alkanolamine salt, or ammonium salt. From the viewpoint of cleaning effect, a sodium salt, potassium salt, or magnesium salt is preferred.

From the viewpoint of the allergen-removing effect, it is also preferred to use, as the cationic surfactant, any one of compounds of the following general formulae (3) to (5) :

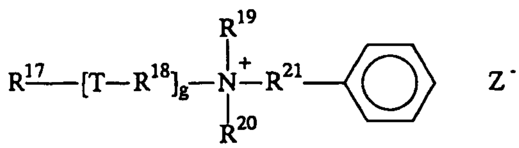

(3)

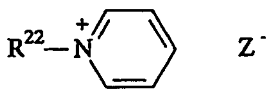

(4)

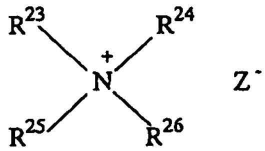

(5)

wherein $R^{17}$ and $R^{22}$ are each an alkyl or alkenyl group having 5 to 16 carbon atoms, preferably 6 to 14 carbon atoms, and are each preferably an alkyl group, $R^{19}$ and $R^{20}$ are each an alkyl or hydroxyalkyl group having 1 to 3 carbon atoms, and T is -COO-, OCO-, -CONH-, -NHCO, or

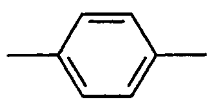

wherein g is a number of 0 or 1, $R^{18}$ is an alkylene group having 1 to 6 carbon atoms, or $-(O-R^{27})_n-$ wherein $R^{27}$ is an ethylene or propylene group, preferably an ethylene group, and n is from 1 to 10, preferably from 1 to 5, as an average number, $R^{21}$ is an alkylene group having 1 to 5 carbon atoms, preferably 2 or 3 carbon atoms, two or more (preferably two) of $R^{23}$, $R^{24}$, $R^{25}$ and $R^{26}$ are alkyl groups having 8 to 12 carbon atoms, and the others are alkyl groups or hydroxyalkyl groups having 1 to 3 carbon atoms, and $Z^-$ is an anion, preferably a halogen ion, or an alkyl sulfate ion having 1 to 3 carbon atoms.

The most preferred cationic surfactant may be the following:

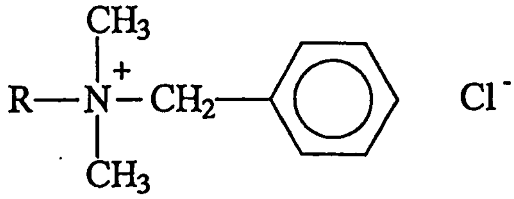

in the formula, R is an alkyl having 8 to 12carbon atoms;

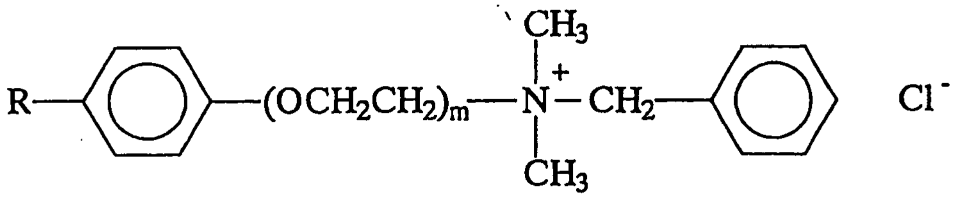

in the formula, R is an alkyl having 6 to 10 carbon atoms, optionally branched, and m is a number of 1 to 5;

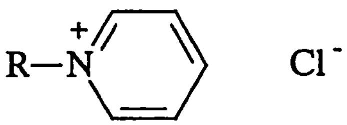

in the formula, R is an alkyl having 8 to 12 carbon atoms.

As an other surfactant, in the present invention, it is preferred that a nonionic surfactant, which does not have the properties of component (c), is comprised in the allergen-removing agent from the viewpoint of appearance and storage stability of the composition. The amount thereof should be limited to the minimum because it may prevent component(c) from solidifying and the allergen may not be removed from the surface.

<Allergen-removing agent>

The allergen-removing agent is in an aqueous solution form in which component (c) and, if necessary, component (d) and other components are dissolved in component (a) and water, which is component (b). In the present invention, component (c) is a particularly important component. Component (c) has a function of collecting or stripping an allergen from the surface of an object. Specifically, when an object is subjected to spraying treatment with the allergen-removing agent or immersing treatment into the allergen-removing agent and then the treated portion is dried by evaporation of the liquid components, the solid component (c) is precipitated on the surface of the object. It appears that at this time, functions as follows are exhibited: component (c) is precipitated while component (c) embraces allergen substances, for example, fine particles which are not easily removed by ordinary cleaning, particularly pollen, spores of fungi, organism such as mites, and dead bodies and dung thereof; or the exfoliation of the allergens from the object to be treated is promoted. By wiping off such precipitated solids together with the allergens with a cloth such as a towel or removing them with a cleaning tool such as a vacuum cleaner, the allergens can easily be removed. The components contained in the allergen-removing agent are used at such concentrations, respectively, that the solid is promoted to precipitate, but the components neither stick nor accumulate on the object's surfaces.

In the present invention, the content of component (a) depends upon the kind of component (c). For example, in the case of using a compound having strong hydrophobicity among components (c1-1) and (c1-2), it is necessary to make the content of component (a) larger than in the case of selecting a compound having superior hydrophilicity as component (c). Component (c2) is desirably blended at a concentration which does not cause saturation. Effects of the ion strength of the other components, should be considered. Component (a) has a low flash point. Thus, when a large amount thereof is used, attention should be paid.

In the present invention, the content of component (a) is from 1 to 70% by mass, preferably from 3 to 60% by mass, and most preferably from 5 to 50% by mass in the allergen-removing agent in order to perform a uniform dissolution

in the composition, promote drying after spraying and application thereof and promote the precipitation of component (c) onto the surface of an object. The water, which is component (b), is contained at a ratio of 30 to 98.999% by mass, preferably 40 to 98% by mass, and most preferably 60 to 96% by mass in the allergen-removing agent. The content of component (c2) is 0.001% or more by mass, preferably 0.005% or more by mass, and 5% or less by mass, preferably 3% or less by mass, the most preferably 1% or less by weight, in the allergen-removing agent.

Particularly, the total of component (a), component (b) and component (c) is preferably from 95 to 100% by mass, more preferably from 97 to 100% by mass, and most preferably from 98 to 100% by mass in the allergen-removing agent.

In component (c2), a molar ratio of inorganic cations/inorganic anions = 0.3 to 3.0 (molar ratio) is preferred and the ratio of from 0.5 to 2.0 is particularly preferred. When component (c1-2) is contained in component (c), the concentration of (c1-2) is preferably 1% or less by mass, more preferably 0.5% or less by mass, and most preferably 0.01 to 0.3% by mass.

In the case component (d) is contained in the allergen - removing agent, the content thereof should be an amount which does not suppress the precipitation of the solid. The content of component (d) in the allergen-removing agent is preferably from 5% or less by mass, more preferably from 0.001 to 3% by mass, and particularly preferably from 0.005 to 1% by mass.

Precipitation of the solid strongly depends on component (d). The more volatile component (d) is, the less influence it has on removal of allergen. For example, methanol has an influence on drying time, but has little influence to component (c). The compounded amounts of the surfactant and the perfume, however, are preferably limited. In the relation between components (c) and (d), the mass ratio of (c) / [(c) + (d)] is preferably 0.15 or more, more preferably 0.2 or more. In the case component (d) is a surfactant or a perfume, it is preferably 0.5 or more, particularly preferably from 0.5 to 0.98

In the case that a perfume (d1) is used in component (d), the content of the organic compounds of component (d) other than component (d1) is desirably 1% or less by mass, more desirably 0.2% or less by mass.

The following Forms A, B, C and D are disclosed as Examples and Reference Examples of the allergen-removing agent.

<Form A> (Reference Example)

An allergen-removing agent comprising the following components (a), (b), (c1-1) and (d),the mass ratio of (c1-1) /[(c1-1)+(d)] thereof being 0.15 or more:

component (a): 1 to 70% by mass of an organic compound which forms with water into an azeotropic mixture, and has an azeotropic temperature with water of less than 100°C at 1013.25 hPa,
component (b): water,
component (c1-1): 0.001 to 5% by mass of an organic compound having a melting point of 25°C or more (provided that the compound is not any surfactant), and
component (d): 0 to 5% by mass of organic compounds of component (a) and component (c1-1)

<Form B> (Reference Example)

An allergen-removing agent comprising the following components (a), (b), (c1) and (d1),the mass ratio of (c1) / [(c1) + (d1)] thereof being from 0.2 to 1, and the content of organic compounds other than (a), (c1) and (d1) being from 0 to 1% by mass:

component (a): 1 to 70% by mass of an organic compound which forms with water into an azeotropic mixture, and has an azeotropic temperature with water of less than 100°C at 1013.25 hPa,
component (b): water, and
component (c1): 0.01 to 5% by mass of an organic compound selected from the following (c1-2) and (c1-3):

component (c1-2): a surfactant which is in a solid state at 25°C at a water content of 5% or less by mass,
component (c1-3): citric acid, succinic acid, tartaric acid, malic acid, fumaric acid, malonic acid, maleic acid, adipic acid, lactic acid, phthalic acid, terephthalic acid, asparagic acid, azelaic acid, glutamic acid, glutaric acid, oxalic acid, glycine, and alkali metal salts or alkali earth metal salts thereof, and
component (d1) : 0 to 5% by mass of a perfume component.

<Form C> (Reference Example)

An allergen-removing agent comprising the following components (a), (b), (c1) and (d1-1), the content of organic compounds other than (a), (c1) and (d1-1) being less than 1% by mass, the mass ratio of component (c1)/[all the organic compounds other than component (a)] being 0.2 or more to less than 1:

component (a): 1 to 70% by mass of an organic compound which forms with water into an azeotropic mixture, and has an azeotropic temperature with water of less than 100°C at 1013.25 hPa,
component (b): water,
component (c1): 0.01 to 5% by mass, preferably 0.01 to 3% by mass, more preferably 0.01 to 1% by mass of one or more organic compounds selected from the following components (c1-1), (c1-2) and (c1-3):

component (c1-1): an organic compound having a melting point of 25°C or more, provided that (c1-2) and (c1-3) are excluded,
component (c1-2): a surfactant which is in a solid state at 25°C and has a water content of 5% or less by mass,
component (c1-3): citric acid, succinic acid, tartaric acid, malic acid, fumaric acid, malonic acid, maleic acid, adipic acid, lactic acid, phthalic acid, terephthalic acid, asparagic acid, azelaic acid, glutamic acid, glutaric acid, oxalic acid, glycine, and alkali metal salts or alkali earth metal salts thereof, and
component (d1-1):0.001 to 5% by mass of one or more compounds selected from jasmonoid, chained sesquiterpene alcohol and chained diterpene alcohol.

<Form D>

An allergen-removing agent comprising the following components (a), (b) and (c2), the molar ratio of (c2-c) / (c2-a) of component (c2) is from 0.3 to 3.0 and the content of inorganic cations other than the (c2-c) and hydrogen ions being less than 0.5% by mass:

component (a) : 1 to 70% by mass of ethanol, 1-propanol or 2-propanol, which form with water an azeotropic mixture, and have an azeotropic temperature with water of less than 100°C at 1013.25 hPa,
component (b): water,
component (c2) :0.01 to 30%, preferably 0.01% to 10%, and more preferably 0.01% to 5% by mass of a cation selected from the following component (c2-c) and an anion selected from the following component (c2-a):

component (c2-c): potassium, sodium and magnesium ions, and
component (c2-a): a sulfate ion and a carbonate ion. Attention should be paid to the setting of the pH of the allergen-removing agent used in the present invention. In the case of a substance which is solid in the state of a salt thereof but is dissolved in acid or alkali, it can happen that a solid is not easily precipitated depending on the pH. In the present invention, therefore, it is preferred to set the pH of the allergen-removing agent at 20°C to 3-9, preferably 4-8. The pH adjuster is preferably a substance constituting component (c). It is preferred to use, as the pH adjuster, the following alone or in combination: acid chemicals, for example, an inorganic acid such as hydrochloric acid or sulfuric acid, and an organic acid such as citric acid, succinic acid, malic acid, fumaric acid, tartaric acid, malonic acid or maleic acid; or alkali chemicals such as sodium hydroxide, potassium hydroxide, sodium carbonate and potassium carbonate. It is particularly preferred to use an acid selected from hydrochloric acid, sulfuric acid and citric acid, or an alkali agent selected from sodium hydroxide and potassium hydroxide. As the alkali agent, ammonia, a derivative thereof, or an amine compound such as monoethanol amine, diethanol amine or triethanol amine may be used to a degree that precipitation of the solid is not prevented. However, these alkali agents are liquid. In the present invention, therefore, it is preferred to use a different alkali agent.

In order to improve the easiness of the treatment of an object with the allergen-removing agent used in the present invention and improve the allergen-removing effect, it is preferred to adjust the viscosity of the allergen-removing agent at 20°C to 15 mPa·sor less, preferably 1 to 10 mpa·s. By the adjustment to such a viscosity, the object can be uniformly treated. Furthermore, drying or the precipitation of component (C) can be promoted.

The allergen-removing agent used in the present invention is applied by spraying onto an object to be treated, or by other treatment of an object to be treated, and then the treated object is dried, thereby precipitating a solid. The solid is generated from component (c). In the case that component (c) is composed of materials of various kinds various solids may be precipitated. A solid having crystal water may be precipitated. The precipitated solid(s) is/are preferably in a form which is easily removed by cleaning. It is preferred to precipitate a material wherein the maximum size of each

solid is from 1 to 100 μm. Even if the individual solids have a size not more than the above-mentioned size, the solids may grow into a large solid by repeated applications. Since it is preferred that the precipitated solid can easily be removed from the object to be treated by cleaning, preferably the solid neither has deliquescence nor adhesiveness. The shape of the precipitated solid is not particularly important. Examples thereof include spherical, polygonal, plate and needle shapes.

<Allergen-removing method>

The present invention provides an allergen-removing method, based on a new idea, of bringing a solution which generates a solid after being dried into contact with an object which an allergen adheres to, and removing, from the object, the allergen together with the precipitated solid, thereby removing the allergen. The precipitated solid is cleaned and removed at last. Examples of the cleaning method include a method of absorbing the solid with a vacuum cleaner, a method of wiping off the solid with a fibrous product or the like, a method of adsorbing the solid with an adhesive film, a method of adsorbing the solid by static electricity, and a method of wiping off the solid with a broom. In the present invention, most preferred are the method of removing the precipitated solid and the allergen with a vacuum cleaner and the method of wiping off the precipitated solid and the allergen with a fibrous product so as to confine them in the fiber. The time from the application or spray of the allergen-removing agent to the cleaning thereof is preferably 10 seconds or more and 60 minutes or less.

When the allergen-removing agent is sprayed, it is preferred to use a trigger type sprayer as a sprayer.

A trigger type sprayer spraying 0.1 to 2.0g, more preferably 0.2 to 1.5g, furthermore preferably 0.3 to 1.0g, at one stroke is preferred. As a container of the trigger type sprayer used in the present invention, a pressure-accumulating type trigger as disclosed in JP(U)-A No. 4-37554 is particularly preferred from the viewpoint of the uniformity of the spray.

As to spraying property, preferred is a trigger type sprayer having the following ability: an area where liquid is sprayed from this spray is spread from 100 to 800 $cm^2$, preferably from 150 to 600 $cm^2$, wherein the liquid being sprayed to an object is vertically placed on the ground from a spot 15 cm apart. In the present invention, a high allergen-removing effect can be obtained by spraying component (c) uniformly onto an object in an amount of 1 to 10 mg, preferably 2 to 5 mg, per 1000 $cm^2$ of the object, and then drying the sprayed components.

After the spraying, allergens can be effectively removed by drying the sprayed components naturally and removing the object with a cleaning tool, for example, a cloth such as a towel, or a vacuum cleaner.

The following will describe formulation examples of the allergen-removing agent used in the Examples and Reference Examples below.

An aqueous composition made of:

(A) one or more selected from ethanol, 1-propanol, and 2-propanol: 3 to 60% mass,
(B) water: balance,
(C) a solid source material selected from the following (1) to (4): 0.05 to 5% by mass (provided that (1) or (2) is not incorporated in the composition in an amount over 1% by mass, especially 0.5% by mass):

  (1) cedrol, 1-menthol, myristyl alcohol, palmityl alcohol, stearyl alcohol, camphene, and thymol,
  (2) saturated alkyl sulfate having an alkyl chain having 14 to 20 carbon atoms, or a salt thereof, and saturated fatty acid soap having 14 to 18 carbon atoms (the salt is preferably an alkali metal salt),
  (3) citric acid, succinic acid, tartaric acid, malic acid, fumaric acid, malonic acid, maleic acid, adipic acid, phthalic acid, terephthalic acid, and alkali metal salts or alkali earth metal salts thereof(preferably alkali metal salts), and
  (4) combination of a cation selected from a potassium ion, a sodium ion and a magnesium ion, and an anion selected from a sulfate ion and a carbonate ion (provided that a combination of only potassium and carbonate ions is excluded).

(D) jasmone, dihydrojasmone, methyl jasmonate, methyl dihydrojasmonate, farnesol, andnerolidol: 0 to 0.1% by mass, and
(E) Other components: 0 to 2.0% by mass.

Furthermore, the composition is charged into a container having a sprayer of a non-aerosol type, particularly a composition charged into the container, which causes precipitation of a solid within 30 minutes under an environment of 23 to 32°C temperature and of 60% RH or less humidity when the spraying operation is performed one time at a position 30 cm apart from an object's surface. The ratio of component (c) to the sum total of component (D) and surfactant (E) is 0.5 to 0.98 by mass.

Examples of object surfaces to which the allergen-removing agent is applied include a carpet, a tatami mat, a cloth sofa, rags, a floorboard, indoor hard surfaces from which an allergen is requested to be removed, bedlines such

as a coverlet, a pillow and a mattress pad, a stuffed toy; and a cloth cushion.

Example

Blend Examples 1-1 to 1-8 [Reference Examples]

Allergen-removing agents shown in Table 1 were prepared. An object prepared in the following manner was treated with each of the allergen-removing agents in the following manner. The allergen-removing effect thereof was then measured. The feel after the treatment was examined. Results are shown in Table 1.

(1) Measurement of the allergen-removing ratio

A used carpet (carpet "Sun Cecil CL-1" made by Sangetsu Co., Ltd.), which had been used in a home for 3 years, was cut into 10 cm square pieces.

Each of the samples was uniformly sprayed, in an amount of 0.3 g, onto each of the cut carpet pieces (using a trigger device used as a starching agent for fabrics, "SMOOTHER" as commercial name, marketed by Kao Corp.) The spray amount was 0.3 g at one stroke. When the liquid was sprayed onto a vertically-placed object surface from a spot 15 cm apart, the area where the liquid was spread was 420 $cm^2$.) The sprayed pieces were dried at room temperature for 30 minutes. Thereafter, the objects on the surface were vacuumed by a vacuum cleaner in which a new paper pack was fitted for one second with 250 W as the vacuum power.

Thereafter, 50 mL of a buffer solution having a pH of 7.4 ± 0.1 (a solution wherein $KH_2PO_4$, NaCl, and $NaH_2PO_4 \cdot 7H_2O$ were dissolved in distilled water to have concentrations of 0.144 g/L, 9.00 g/L and 0.795 g/L, respectively, the solution being referred to as PBS hereinafter) was used to extract allergens collected into the paper pack (this extracted liquid being called the removed-allergen extract). Allergens remaining in the carpet were extracted with 50 mL of PBS (this extracted liquid being called the remaining-allergen extract).

In the respective extracts, the concentration of Der f II (allergens contained in Dermatoph goides fanine) was quantitatively determined by sandwich ELISA. The sandwich ELISA was performed as follows.

1. Monoclonal antibody 15E11 (made by Seikagaku Corporation) was diluted into a concentration of 2 μg/mL with PBS, and 50 μL of the diluted antibody was poured into each well in a microplate (ELISA PLATE H TYPE, made by Sumitomo Bakelite). The microplate was allowed to stand still at room temperature for 2 hours.
2. The plate was washed with PBS 3 times.
3. Into each well was poured 200 μL of PBS containing 1% BSA (Block Ace, made by Dainippon Pharmaceutical Co. , Ltd.) and then the plate was allowed to stand still at room temperature for 1 hour to perform blocking.
4. The plate was washed three times with PBS containing 0.05% by mass of Tween 20 (SIGMA) (hereinafter referred to as T-PBS).
5. As standards, rDer f II (made by Seikagaku Corporation) was diluted $2^n$ times from 0.3 μg/mL, using T-PBS in 9 tubes, and then 50 μL of each of the diluted solutions was poured into each of the wells. As a negative control, a well to which 50 μL of T-PBS was added instead of rDer f II was prepared. The sample to be measured was appropriately diluted with T-PBS, and 50 μL of the diluted sample was poured into each of the wells. The plate was allowed to stand still at room temperature for 2 hours.
6. The plate was washed 3 times with T-PBS.
7. 50 μL of HRP-labeled 13A4 (made by Seikagaku Corporation) having an appropriate concentration was poured into each of the wells, and the plate was allowed to stand still at room temperature for 2 hours.
8. The plate was washed 3 times with T-PBS.
9. A color-developing kit T (made by Sumitomo Bakelite) for peroxidase was used to develop color. First, 0.1 mL of a substrate liquid was added to 10 mL of a color-developing agent, and these were blended to prepare a color developing solution. 100 μL of this color developing solution was poured into each of the wells, to develop color at room temperature. Thereafter, 100 μL of a stop solution was poured into each of the wells to stop the reaction. The absorbance at 450 nm of each of the solutions was measured with a plate reader.
10. The Der f II concentration of the sample to be measured was calculated, using a calibration curve obtained from the absorbances of the standards.

About the resultant Der f II concentration, the Der f II concentration of the removed-allergen extraction is represented by a and the Der f II concentration of the remaining-allergen extraction is represented by b. At this time, the allergen-removing ratio R by the absorption with the vacuum cleaner is defined by the following equation:

$$R = a/(a + b) \times 100 \qquad (\%)$$

The test was performed 5 times about each of the samples, and the average value of the resultant 5 allergen removing ratios was used as the allergen-removing ratio of each of the samples.

(2) Method of evaluating the feel

A carpet "Sun Cecil CL-101" made by Sangetsu Co., Ltd was cut into 10 cm square pieces. The pieces were used as test pieces. The formulation solution was sprayed onto each of the test pieces at 0.1 cc/100 $cm^2$ and was sufficiently dried. The spraying and the drying were repeated 50 times. The resultant was used as a sample. Ten panelists performed functional evaluations according to the following criteria on the standard of the test piece subjected to no treatment. The average value thereof was obtained.

4: Better feel than the standard
3: Equivalent to the standard
2: Slightly poorer feel than the standard
1: Evidently poorer feel than the standard

Table 1

| Allergen-removing agent | | | Note | Blend example 1-1 | 1-2 | 1-3 | 1-4 | 1-5 | 1-6 | 1-7 | 1-8 |
|---|---|---|---|---|---|---|---|---|---|---|---|
| Blend component (% by mass) | (a) | Ethanol | Azeotropic temperature78.2°C | 50 | | | 15 | 50 | 50 | 50 | |
| | | Isopropanol | Azeotropic temperature80.1°C | | 50 | 50 | | | | | |
| | (c) (c1-1) | Cedrol | Melting point86°C | | | | | | | 10 | |
| | | Camphene | Melting point51°C | | | 0.1 | | | 0.1 | | |
| | | Myristyl alcohol | Melting point38°C | 0.1 | | | 0.1 | | | | |
| | | Thymol | Melting point51°C | | 0.1 | | | | | | |
| | (d) | Decanol | Melting point7°C | | | | | 0.1 | | | |
| | | Phenoxyethyl alcohol | Melting point13°C | | | | | | | | |
| | | Farnesol | — | | 0.05 | | | | 0.7 | 0.15 | |
| | | Methyl dihydrojasmonate | — | 0.05 | | | 0.02 | | | | |
| | | Ajowan oil[containing 47% by mass of thymol ((c1-1)component] | — | | | | | | | | |
| | | Nonion | — | 0.05 | 0.05 | 0.05 | 0.3 | 0.05 | 0.05 | 0.05 | |
| | | Anion | — | | | | 0.1 | | | | |
| | (b) | Water and pH adjuster | | Balance | Balance | Balance | Balance | Balance | Balance | Balance | 100 |
| | | Total | | 100 | 100 | 100 | 100 | 100 | 100 | 100 | 100 |
| | | pH(20°C) | | 7 | 7 | 7 | 7 | 7 | 7 | 7 | 7 |
| Allergen-removing effect (%) | | | | 58 | 55 | 53 | 52 | 25 | 38 | 51 | 18 |
| Feel evaluation | | | | 3.0 | 3.1 | 3.0 | 3.0 | 2.2 | 1.5 | 1.2 | 3.0 |

In Table 1, "Nonion" represents alkylpolyglycoside (alkyl group: carbon number of 10/carbon number of 12 = 4/6 (mass ratio), and glucose average condensation degree: 1.3), and "Anion" represents alkenylsuccicnic acid having an alkenyl group having 12 carbon atoms. Solubilities of cedorol, camphene, myristyl alcohol and thymol in water are all 0.02 g/100 g or less. All solubilities thereof in component (a) are 0.05 g/100 g or more. All hygroscopic degrees are within the range of 0 to 0.5.

The removing allergen ratio of carpet treated with only the vacuum cleaner without using any composition described in Table 1 was 21%, and the feel evaluation thereof was 3.5.

Blend Examples 2-0 to 2-22 [Reference Examples]

Allergen-removing agents shown in Tables 2 and 3 were prepared, and in the same manner as about Blend Examples 1-1 etc. , the allergen-removing effect and the feel thereof were measured. The results are shown in Tables 2 and 3.

Table 2

| | | | Blend example | | | | | | | | Reference example |
|---|---|---|---|---|---|---|---|---|---|---|---|
| | | | 2-1 | 2-2 | 2-3 | 2-4 | 2-5 | 2-6 | 2-7 | 2-8 | 2-0 |
| Allergen-removing agent: Blend component (% by mass) | (a) | Ethanol | | 30 | | 30 | 15 | | 15 | 15 | |
| | (a) | Isopropanol | 15 | | 15 | | | 15 | | | |
| | (c) (c1-2) | Sodium myristyl sulfate | 0.1 | | | | | 0.1 | | | 50 |
| | (c) (c1-2) | Sodium palmityl sulfate | | 0.1 | | | | | | | 50 |
| | (c) (c1-2) | Sodium myristate | | | 0.1 | | | | | | |
| | (c) (c1-2) | Sodium palmitate | | | | 0.1 | | | | | |
| | (c) (c1-2) | Polyoxyethylene lauryl ether (EOp=40) | | | | | 0.1 | | | | |
| | (c) | Polyoxyethylene lauryl ether (EOp=10) | | | | | | | 0.1 | | |
| | (d) | Decanoic acid | | | | | | | | 0.1 | |
| | (d) (d1) | Farnesol | 0.02 | 0.02 | 0.02 | 0.02 | | 0.3 | 0.02 | 0.02 | |
| | (d) (d1) | Metyl jasmonate | | | | | 0.02 | | | | |
| | (b) | Water and pH adjuster | Balance | Balance | Balance | Balance | Balance | Balance | Balance | Balance | — |
| | | Total | 100 | 100 | 100 | 100 | 100 | 100 | 100 | 100 | 100 |
| | (c)/[(c)+(d1)]mass ratio* | | 0.83 | 0.83 | 0.83 | 0.83 | 0.83 | 0.25 | 0.83 | 0.83 | — |
| | pH(20°C) | | 7.8 | 7.8 | 7.8 | 7.8 | 6.7 | 7.8 | 6.4 | 6.8 | — |
| Allergen-removing effect(%) | | | 55 | 59 | 56 | 58 | 56 | 54 | 22 | 28 | 31 |
| Feel evalustion | | | 3.2 | 3.4 | 2.7 | 2.8 | 2.7 | 2.9 | 2.2 | 2.2 | 1.4 |

Table 3

| | | | | Blend example | | | | | | | | | | | | | |
|---|---|---|---|---|---|---|---|---|---|---|---|---|---|---|---|---|---|
| | | | | 2-9 | 2-10 | 2-11 | 2-12 | 2-13 | 2-14 | 2-15 | 2-16 | 2-17 | 2-18 | 2-19 | 2-20 | 2-21 | 2-22 |
| Allergen-removing agent | Blend component (% by mass) | (a) | Ethanol | | 30 | | 30 | 50 | 50 | 50 | 50 | 50 | 50 | 50 | 50 | 50 | 50 |
| | | | Isopropanol | 30 | | 30 | | | | | | | | | | | |
| | | (c) (c1-3) | Succinic acid | 0.1 | | | | | | | | | | | | | |
| | | | Malic acid | | 0.1 | | | | | | | | | | | | |
| | | | Fumaric acid | | | 0.1 | | | | | | | | | | | |
| | | | Malonic acid | | | | 0.1 | | | | | | | | | | |
| | | | Maleic acid | | | | | 0.1 | | | | | | | | | |
| | | | Adipic acid | | | | | | 0.1 | | | | | | | | |
| | | | Lactic acid | | | | | | | 0.1 | | | | | | | |
| | | | Terephthalic acid | | | | | | | | 0.1 | | | | | | |
| | | | Asparagic acid | | | | | | | | | 0.1 | | | | | |
| | | | Azelaic acid | | | | | | | | | | 0.1 | | | | |
| | | | Glutamic acid | | | | | | | | | | | 0.1 | | | |
| | | | Glutaric acid | | | | | | | | | | | | 0.1 | | |
| | | | Oxalic acid | | | | | | | | | | | | | 0.1 | |
| | | | Glycine | | | | | | | | | | | | | | 0.1 |
| | | (d1) | Farnesol | 0.02 | 0.02 | 0.02 | 0.02 | | | | | 0.02 | 0.02 | 0.02 | 0.02 | | |
| | | | Methyl jasmonate | | | | | 0.02 | 0.02 | | | | | | | 0.02 | 0.02 |
| | | | Ajowan oil | | | | | | | 0.02 | 0.02 | | | | | | |
| | | (b) | Water and pH adjuster | Balance | Balance | Balance | Balance | Balance | Balance | Balance | Balance | Balance | Balance | Balance | Balance | Balance | Balance |
| | | Total | | 100 | 100 | 100 | 100 | 100 | 100 | 100 | 100 | 100 | 100 | 100 | 100 | 100 | 100 |
| | (c)/[(c)+(d1)]mass ratio* | | | 0.83 | 0.83 | 0.83 | 0.83 | 0.83 | 0.83 | 0.90 | 0.90 | 0.83 | 0.83 | 0.83 | 0.83 | 0.83 | 0.83 |
| | pH(20°C) | | | 7.8 | 7.8 | 7.8 | 7.8 | 6.7 | 7.9 | 7.6 | 6.8 | 6.4 | 6.8 | 7.8 | 7.2 | 7.3 | 7.5 |
| Allergen-removing effect(%) | | | | 52 | 53 | 50 | 51 | 51 | 53 | 50 | 50 | 51 | 50 | 51 | 52 | 51 | 50 |
| Feel evaluation | | | | 3.1 | 3.0 | 3.1 | 3.1 | 3.1 | 3.1 | 3.1 | 3.1 | 3.0 | 3.0 | 3.0 | 3.1 | 3.1 | 3.1 |

* In Tables 2 and 3, mass ratio (c)[(c) + (d1)] means the mass ratio of [(c1-2) + (c1-3)]/[(c1-2) + (c1-3) + (d1)].

In the tables, ethanol has an azeotropic temperature with water of 78.2°C, isopropanol has an azeotropic temperature of 80.1°C. All of sodium myristyl sulfate, sodium palmityl sulfate, sodium myristate, sodium palmitate and polyoxyethylene lauryl ether (EOp = 40) are in a solid state at 25°C and 35°C at a water content of 5% by mass. All of polyoxyethylene lauryl ether (EOp = 10) and decanoic acid were in a liquid state at 25°C and 35°C at a water content of 5% by mass. The EOp is an ethylene oxide average addition mol number (the same matter is applied correspondingly to the following). All of components (c1-2) in table 2 had a hygroscopic degree within the range of 0 to 0.5. In Tables 2 and 3, the adjustment of pH was performed with sulfuric acid and/or sodium hydroxide.

In Table 2, Reference Example 2-0 is an example wherein the effect of using powder, as it was, without preparation into an aqueous solution was examined. Component (c1-2) shown in Reference Example 2-0 was pulverized and classified into a size of 50 to 125 μm with sieves, and 5 g of the resultant powder was uniformly sprinkled on the cut carpet. After 30 minutes, the powder was absorbed with a vacuum cleaner. In the same way as the above except this operation, the allergen-removing effect and the feel were evaluated.

The allergen-removing ratio of the carpet treated with only the vacuum cleaner without using any composition described in Tables 2 and 3 was 21%, and the feel evaluation thereof was 3.5.

Blend Examples 3-1 to 3-4 and Reference Examples 3-5 to 3-13

Allergen-removing agents shown in Table 4 were prepared, and in the same manner as with Blend Examples 1-1 etc., the allergen-removing effects thereof were measured, and the feel after the treatment was examined. The results are shown in Table 4.

Table 4

| | | Blend example | | | | | | | | | | | | Reference example |
|---|---|---|---|---|---|---|---|---|---|---|---|---|---|---|
| | | 3-1 | 3-2 | 3-3 | 3-4 | 3-5 | 3-6 | 3-7 | 3-8 | 3-9 | 3-10 | 3-11 | 3-12 | 3-13 |
| Allergen-removing agent / Blend component (% by mass) (a) | Ethanol | 50.0 | 15.0 | 15.0 | 15.0 | 50.0 | 50.0 | 15.0 | 15.0 | – | – | – | 15.0 | – |
| (a) | Isopropanol | – | – | – | – | – | – | – | – | 15.0 | 15.0 | 15.0 | – | – |
| (c2) | Sodium sulfate | 1.0 | – | – | 2.0 | – | – | – | – | – | – | – | – | – |
| (c2) | Magnesium sulfate | – | 1.0 | – | – | – | – | – | – | – | – | – | – | 92.0 |
| (c2) | Sodium carbonate | – | – | 1.0 | – | – | – | – | – | – | – | – | – | – |
| | Magnesium chloride | – | – | – | – | 1.0 | – | – | – | – | – | – | 3.0 | – |
| | Iron sulfate | – | – | – | – | – | – | 1.0 | – | – | – | – | 3.0 | – |
| | Potassium carbonate | – | – | – | – | – | – | – | – | 1.0 | – | – | – | – |
| | Sodium dihydrogenphosphate | – | – | – | – | – | – | – | – | – | 1.0 | – | – | – |
| | Sodium hydride | – | – | – | – | – | – | – | – | – | – | 1.0 | – | – |
| | Zinc chloride | – | – | – | – | – | 1.0 | – | – | – | – | – | – | – |
| | Calcium chloride | – | – | – | – | – | – | – | 1.0 | – | – | – | – | – |
| (c) (c1-1) | Cedrol | 0.1 | – | – | – | – | – | – | – | – | – | – | – | 5.0 |
| (c) (c1-1) | Camphene | 0.01 | 0.01 | – | – | 0.01 | 0.01 | 0.01 | 0.01 | 0.01 | 0.01 | 0.01 | 0.01 | – |
| (c) (c1-1) | Thymol | 0.01 | 0.01 | – | – | 0.01 | 0.01 | 0.01 | 0.01 | 0.01 | 0.01 | 0.01 | 0.01 | – |
| (d) (d1) | Farnesol | – | 0.1 | – | – | 0.1 | 0.1 | 0.1 | 0.1 | 0.1 | 0.1 | 0.1 | 0.1 | – |
| (d) (d1) | Methyl jasmonate | – | – | 0.1 | 0.1 | – | – | – | – | – | – | – | – | 2.0 |
| (d) (d1) | Ajowan oil | – | – | 0.02 | – | – | – | – | – | – | – | – | – | 1.0 |
| (d) | Nonionic surfactant | – | 0.1 | 0.1 | – | – | – | 0.1 | 0.1 | 0.1 | 0.1 | 0.1 | 0.1 | – |
| (d) | Anionic surfactant | – | 0.05 | 0.05 | – | – | – | 0.05 | 0.05 | 0.05 | 0.05 | 0.05 | 0.05 | – |
| (b) | Ion-exchange water | Balance | Balance | Balance | Balance | Balance | Balance | Balance | Balance | Balance | Balance | Balance | Balance | – |
| | Total | 100 | 100 | 100 | 100 | 100 | 100 | 100 | 100 | 100 | 100 | 100 | 100 | 100 |
| | pH(20℃) | 7.2 | 6.8 | 9.0 | 6.8 | 6.8 | 6.2 | 4.1 | 6.7 | 10.5 | 4.5 | 13.0 | 6.8 | – |
| | Total (% by mass) of (c2-c)+(c2-a) | 1.0 | 1.0 | 1.0 | 2.0 | 0.25 | 0 | 0.63 | 0 | 0.43 | 0.19 | 0.56 | 2.7 | 92 |
| | (c2-c)/(c2-a) (molar ratio) | 2/1 | 1/1 | 2/1 | 2/1 | – | 0/0 | – | 0/0 | – | – | – | 1.6/1 | 1/1 |
| | Total(% by mass) of cationic ions from which (c2-c) and hydrogen ions were excluded | 0 | 0 | 0 | 0 | 0 | 0.48 | 0.37 | 0.36 | 0.57 | 0 | 0 | 1.1 | 0 |
| Allergen-removing effect(%) | | 43 | 45 | 38 | 58 | 19 | 21 | 19 | 20 | 16 | 18 | 20 | 21 | 22 |
| Feel evaluation | | 3.1 | 3.3 | 3.2 | 3.0 | 2.2 | 2.4 | 2.3 | 2.4 | 2.0 | 2.3 | 2.1 | 2.4 | 2.0 |

In Table 4, nonionic surfactant represents alkylpolyglycoside (alkyl group: carbon number of 10/carbon number of 12 = 4/6 (mass ratio), and glucose average condensation degree: 1.3), and anion surfactant represents alkenylsuccicnic acid having an alkenyl group having 12 carbon atoms. Compound (c2) is a compound which becomes a supply source of component (c).

In Table 4, Reference Example 3-13 is an example wherein the effect of using powder, as it was, without preparation into an aqueous solution was examined. The composition (in a solid form) described in Reference Example 3-13 was classified into a size of 500 to 1000 $\mu$m with sieves, and 5 g of the resultant powder was uniformly sprinkled on the cut carpet. After 30 minutes, the powder was vacuumed with a vacuum cleaner. In the same way as above except this, the allergen-removing effect and the feel were measured.

The allergen-removing ratio of the carpet treated with only the vacuum cleaner without using any composition described in Table 4 was 21%, and the feel evaluation thereof was 3.5.

Solubilities at 25°C of sodium sulfate, magnesium sulfate and sodium carbonate are 21.9%, 26.7% and 22.7%, respectively. All hygroscopic property tests defined in the specification of the present application are within the range of

0 to 0.5.

Blend Examples 4-0 to 4-30 (Reference Examples)

Allergen-removing agents shown in Tables 5 to 7 were prepared, and in the same manner as with Blend Example 1-1 etc., the allergen-removing effect thereof was measured, and the feel after the treatment was examined. The durability of the allergen-removing effect was evaluated by the following method. The results are shown in Tables 5 to 7. In Tables 5 to 7, the adjustment of pH was performed with sulfuric acid and/or sodium hydroxide.

* Method of evaluating the durability of the allergen-removing effect

A used carpet (carpet "Sun Cecil CL-1" made by Sangetsu Co., Ltd.), which had been used at a home for 3 years, was cut into 10 cm square pieces.

Among the cut carpet pieces, two pieces adjacent to each other were used as one pair, and 0.3 g of the allergen-removing agent was uniformly sprayed on the two pieces [the same trigger container as described in "(1) Measurement of the allergen-removing ratio" of Blend Example 1-1 etc.], and dried at room temperature for 30 minutes. Thereafter, the two pieces were vacuumed with a vacuum cleaner in which a new paper pack was fitted for 1 second with 250 W as the vacuum power.

In one of the pair of carpet pieces, allergens therein were quantitatively determined by vacuuming them with a vacuum cleaner, immediately extracting the remaining on the carpet with 50 ml of PBS and determining the allergens of the extracted solution according to the method used in "(1) Measurement of the allergen-removing ratio", shown in Blend Example 1-1 etc. to obtain "allergen amount immediately after absorption". The other carpet was allowed to stand still at 25°C at 80% RH humidity of the environment for 3 days. "Allergen amount after 3 days" was then obtained similarly by extracting allergens remaining thereon and determining them quantitatively. Calculated from the data, the ratio of the "allergen amount after 3 days"/the "allergen amount immediately after the absorption" was obtained. On the basis of this numerical value, the durability was evaluated. The durability of the allergen-removing effect is better when the numerical value is smaller.

Table 5

| | | | | Blend example | | | | | | | | | Reference example |
|---|---|---|---|---|---|---|---|---|---|---|---|---|---|
| | | | | 4-1 | 4-2 | 4-3 | 4-4 | 4-5 | 4-6 | 4-7 | 4-8 | 4-9 | 4-0 |
| Allergen-removing effect | Blend component (% by mass) | (d) (d1-1) | Cis-jasmone | 0.01 | 0.01 | | | | 0.01 | | 0.01 | | |
| | | (d) (d1-1) | Phytol | | | 0.01 | | 0.01 | | | 0.01 | | |
| | | (d) (d1-1) | Nerolidol | | | | 0.01 | | | 0.01 | | | |
| | | (c) (c1-2) | Sodium myristyl sulfate | 0.1 | | | | | | | | 0.1 | 50 |
| | | (c) (c1-2) | Sodium palmityl sulfate | | 0.1 | | | | | | | | |
| | | (c) (c1-2) | Sodium myristate | | | 0.1 | | | | | | | |
| | | (c) (c1-2) | Sodium palmitate | | | | 0.1 | | | | | | 30 |
| | | (c) (c1-2) | Polyoxyethylene lauryl ether (EOp=40) | | | | | 0.1 | | | | | |
| | | (c) (c1-3) | Citric acid | | | | | | 0.1 | | | | |
| | | (c) (c1-3) | Phthalic acid | | | | | | | 0.1 | | | 20 |
| | | (c) (c1-3) | Tartaric acid | | | | | | | | 0.1 | | |
| | | (a) | Ethanol | | | | | 50 | 50 | 50 | 50 | 50 | |
| | | (a) | Isopropanol | 50 | 50 | 50 | 50 | | | | | | |
| | | (d) | Carbon | | | | | 0.01 | | | | 0.01 | |
| | | (d) | p-t-butylcyclohexyl acetate | | | | | | 0.01 | | 0.01 | | |
| | | (d) | Linalool | | | | | | | 0.01 | | | |
| | | (b) | Water and pH adjuster | Balance | Balance | Balance | Balance | Balance | Balance | Balance | Balance | Balance | – |
| | | Total | | 100 | 100 | 100 | 100 | 100 | 100 | 100 | 100 | 100 | 100 |
| | (c)/[organic compound other than component (a)] | | | 0.91 | 0.91 | 0.91 | 0.91 | 0.83 | 0.83 | 0.83 | 0.77 | 0.91 | 1 |
| | pH(20°C) | | | 7.8 | 7.6 | 7.8 | 7.8 | 6.2 | 6.6 | 6.4 | 6.6 | 7.8 | - |
| Allergen -removing effect(%) | | | | 51 | 58 | 54 | 58 | 49 | 49 | 51 | 50 | 50 | 28 |
| Durability of allergen-removing effect | | | | 1.0 | 1.1 | 1.2 | 1.2 | 1.4 | 1.4 | 1.4 | 1.1 | 1.8 | 1.8 |
| Feel evaluation | | | | 3.2 | 3.0 | 3.2 | 3.2 | 3.1 | 3.0 | 3.0 | 3.1 | 3.1 | 2.5 |

In Table 5, ethanol has an azeotropic temperature with water of 78.2°C, and isopropanol has that of 80.1°C. Methanol makes no azeotropic mixture with water.

Sodium myristyl sulfate, sodium palmityl sulfate, sodium myristate, sodium palmitate and polyoxyethylene lauryl ether (EOp40) are all in a solid state at 25°C and 35°C and at a water content of 5% by mass. The hygroscopic degree of all component (c1-2) ranges between 1 and 1.5.

In Table 5, Reference Example 4-0 was an example wherein the effect of using powder, as it was, without preparation into an aqueous solution was examined. In the manner of treatment thereof, component (c1-2) and component (c1-3) described in Reference Example 4-0 were classified into a size of 50 to 125 μm with sieves, and 5 g of the resultant powder was uniformly sprinkled on the cut carpet. After 30 minutes, the powder was vaccumed with a vacuum cleaner.

In the same way as the above except this operation, evaluations of the allergen-removing effect, the durability and the feel were made.

The allergen-removing ratio of the carpet vacuumed merely with the vacuum cleaner without using any treating agent was 21%, and the feel evaluation thereof was 3.5.

Table 6

| | | | | Note | Blend example 4-10 | 4-11 | 4-12 | 4-13 | 4-14 | 4-15 | 4-16 |
|---|---|---|---|---|---|---|---|---|---|---|---|
| Allergen-removing agent | Blend component (% by mass) | (d) (d1-1) | Dihydrojasmone | Melting point : 20°C or less | 0.1 | | | | | 0.1 | |
| | | | Nerolidol | Melting point : 20°C or less | | 0.1 | | 0.1 | | | |
| | | | Phytol | Melting point : 20°C or less | | | 0.1 | | 0.1 | | |
| | | (c) (c1-1) | Cedrol | Melting point 86°C | 0.1 | | | | | | |
| | | | Camphene | Melting point 51°C | | 0.1 | | | | 0.1 | |
| | | | Myristyl alcohol | Melting point 38°C | | | 0.1 | | | | |
| | | | Palmityl alcohol | Melting point 54°C | | | | 0.1 | | | |
| | | | Thymol | Melting point 51°C | | | | | 0.1 | | |
| | | (a) | Ethanol | Azeotropic temperature 78.2°C | 50 | 50 | 50 | | | 50 | 50 |
| | | | Isopropanol | Azeotropic temperature 80.1°C | | | | 50 | 50 | | |
| | | (d) | Methylphenyl carbinol | Melting point 20°C | | | | | | 0.1 | |
| | | | Phenoxyethyl alcohol | Melting point 13°C | | | | | | 0.1 | 0.1 |
| | | | Carbon | Melting point : 20°C or less | | | | 0.1 | | | |
| | | | p-t-butylcyclohexyl acetate | Melting point : 20°C or less | | | | | 0.1 | | |
| | | | Linalool | Melting point : 20°C or less | | | | | | 0.1 | 0.1 |
| | | (b) | Water and pH adjuster | | Balance | Balance | Balance | Balance | Balance | Balance | Balance |
| | | Total | | | 100 | 100 | 100 | 100 | 100 | 100 | 100 |
| | (c-1)/[(c1-1)+(d)] | | | | 0.5 | 0.5 | 0.5 | 0.33 | 0.33 | 0.2 | — |
| | pH(20°C) | | | | 7.2 | 7.1 | 7.4 | 7.2 | 7.1 | 7.2 | 7.2 |
| Allergen-removing effect (%) | | | | | 58 | 55 | 54 | 56 | 52 | 45 | 28 |
| Durability of allergen-removing effect | | | | | 1.0 | 1.1 | 1.2 | 1.4 | 1.5 | 1.4 | 1.9 |
| Feel evaluation | | | | | 3.0 | 3.1 | 3.0 | 3.1 | 3.0 | 2.2 | 2.7 |

In Table 6, all solubilities of cedrol, camphene, myristyl alcohol, palmityl alcohol and thymol in water (the manner of measuring them being as described above) were 0.3 g/100 g or less, and all solubilities thereof in component (a) (the manner of measuring them being as described above) were 5 g/100 g or more. All hygroscopic degrees thereof were within the range of 1 to 1.5.

Table 7

| | | | | Blend example | | | | | | | | | | | | | |
|---|---|---|---|---|---|---|---|---|---|---|---|---|---|---|---|---|---|
| | | | | 4-17 | 4-18 | 4-19 | 4-20 | 4-21 | 4-22 | 4-23 | 4-24 | 4-25 | 4-26 | 4-27 | 4-28 | 4-29 | 4-30 |
| Allergen-removing agent | Blend component (% by mass) | (d) (d1-1) | Cis-jasmone | 0.01 | 0.01 | | | | 0.01 | | 0.01 | 0.01 | | | 0.01 | | |
| | | | Phytol | | | 0.01 | | 0.01 | | | 0.01 | | 0.01 | | | | 0.01 |
| | | | Nerolidol | | | | 0.01 | | | 0.01 | | | | 0.01 | | 0.01 | |
| | | (c) (c1-1) | Succinic acid | 0.1 | | | | | | | | | | | | | |
| | | | Malic acid | | 0.1 | | | | | | | | | | | | |
| | | | Fumaric acid | | | 0.1 | | | | | | | | | | | |
| | | | Mlonic acid | | | | 0.1 | | | | | | | | | | |
| | | | Maleic acid | | | | | 0.1 | | | | | | | | | |
| | | | Adipic acid | | | | | | 0.1 | | | | | | | | |
| | | | Lactic acid | | | | | | | 0.1 | | | | | | | |
| | | | Terephthalic acid | | | | | | | | 0.1 | | | | | | |
| | | | Asparagic acid | | | | | | | | | 0.1 | | | | | |
| | | | Azelaic acid | | | | | | | | | | 0.1 | | | | |
| | | | Glutamic acid | | | | | | | | | | | 0.1 | | | |
| | | | Glutaric acid | | | | | | | | | | | | 0.1 | | |
| | | | Oxalic acid | | | | | | | | | | | | | 0.1 | |
| | | | Glycine | | | | | | | | | | | | | | 0.1 |
| | | (a) | Ethanol | | | | | 50 | 50 | 50 | 50 | | 50 | 50 | | | 50 |
| | | | Isopropanol | 50 | 50 | 50 | 50 | | | | | 50 | | | 50 | 50 | |
| | | (d) | Carbon | | | | | 0.01 | | | | 0.01 | 0.01 | 0.01 | | | 0.01 |
| | | | p-t-butylcyclohexyl acetate | | | | | | 0.01 | | 0.01 | | | 0.01 | | | |
| | | | Linalool | | | | | | | 0.01 | | 0.01 | | | 0.01 | 0.01 | |
| | | (b) | Water and pH adjuster | Balance | Balance | Balance | Balance | Balance | Balance | Balance | Balance | Balance | Balance | Balance | Balance | Balance | Balance |
| | | Total | | 100 | 100 | 100 | 100 | 100 | 100 | 100 | 100 | 100 | 100 | 100 | 100 | 100 | 100 |
| | (c1-1)/[(c1-1)+(d)] | | | 0.91 | 0.91 | 0.91 | 0.91 | 0.83 | 0.83 | 0.83 | 0.77 | 0.77 | 0.83 | 0.77 | 0.83 | 0.83 | 0.83 |
| | pH (20°C) | | | 7.8 | 7.6 | 7.8 | 7.8 | 6.2 | 6.6 | 6.4 | 6.6 | 6.8 | 6.4 | 6.4 | 7.2 | 7.3 | 7.8 |
| Allergen-removing effect (%) | | | | 50 | 49 | 51 | 51 | 50 | 49 | 50 | 50 | 49 | 50 | 49 | 51 | 51 | 49 |
| Durability of allergen-removing effect | | | | 1.2 | 1.2 | 1.2 | 1.2 | 1.3 | 1.3 | 1.2 | 1.2 | 1.3 | 1.4 | 1.3 | 1.4 | 1.2 | 1.2 |
| Feel evaluation | | | | 3.0 | 3.1 | 3.1 | 3.0 | 3.0 | 3.1 | 3.0 | 3.0 | 3.0 | 3.1 | 3.2 | 3.1 | 3.0 | 3.1 |

Blend Examples 5-1 to 5-7 and 5-9; Reference Example 5-8.

Allergen-removing agents of Blend Examples 5-1 to 5-7 shown in Table 8 were prepared, and then in the same way as in Blend Example 1-1 the allergen-removing effect thereof was measured. An excellent allergen-removing effect was shown. In Table 8, it is added that adjustment of pH was made with sulfuric acid and/or sodium hydroxide.

Table 8

| | | | | | Blend example | | | | | | | | |
|---|---|---|---|---|---|---|---|---|---|---|---|---|---|
| | | | | | 5-1 | 5-2 | 5-3 | 5-4 | 5-5 | 5-6 | 5-7 | 5-8 | 5-9 |
| Allergen-removing agent | Blend component (% by mass) | (a) | | Ethanol | 50 | 50 | 50 | — | — | — | 40 | 60 | 15 |
| | | | | Isopropanol | — | — | — | 50 | 50 | 50 | 10 | | |
| | | (c) | (c1-1) | Cedrol | 0.08 | 0.08 | 0.08 | — | — | — | 0.03 | 0.8 | |
| | | | | Myristyl alcohol | — | — | — | 0.02 | 0.02 | 0.02 | 0.02 | | |
| | | | (c1-2) | Sodium myristate | 0.05 | — | — | 0.05 | — | — | 0.03 | | |
| | | | | Sodium myristyl sulfate | — | 0.06 | 0.06 | — | 0.06 | 0.06 | 0.05 | | |
| | | | (c1-3) | Citric acid | 0.1 | 0.1 | — | 0.1 | 0.1 | — | 0.05 | | |
| | | | | Phthalic acid | — | — | 0.1 | — | — | 0.1 | 0.05 | | |
| | | | (c2) | Sodium sulfate | 0.8 | — | 0.8 | 0.8 | — | 0.8 | 0.4 | | 1.6 |
| | | | | Magnesium sulfate | — | 0.8 | — | — | 0.8 | — | 0.4 | | |
| | | (d) | (d1) | Methyl jasmonate | 0.06 | 0.06 | — | 0.06 | 0.06 | — | 0.03 | 0.06 | 0.06 |
| | | | | Farnesol | — | — | 0.06 | — | — | 0.06 | 0.03 | | |
| | | (b) | | Ion-exchange water | Balance | Balance | Balance | Balance | Balance | Balance | Balance | Balance | Balance |
| | | Total | | | 100 | 100 | 100 | 100 | 100 | 100 | 100 | 100 | 100 |
| | pH (20°C) | | | | 7 | 7 | 7 | 7 | 7 | 7 | 7 | 7 | 7 |

## Claims

1. A method of removing allergens, with an allergen-removing agent, which comprises bringing a liquid composition which generates a solid by removing liquid components thereof into contact with an object to be treated in which an allergen is present and drying the contacted place to form a precipitated solid and removing, from the object, the allergen together with the precipitated solid,
the allergen-removing agent comprising the following component (a), component (b) and component (c2): wherein component (a) at least one compound selected from the group consisting of ethanol, 1-propanol and 2-propanol,
component (b): water and
component (c2): a solid source substance which is soluble in at least one of component (a) and component (b) and generates a solid by evaporation of liquid components of the allergen-removing agent,
being a combination of an inorganic cation with an inorganic anion, the inorganic cation being at least one selected from the group of alkali metal ions and alkaline earth metal ions, the inorganic anion being at least one selected from the group of a sulfate ion, a carbonate ion and a phosphate ion,
the allergen-removing agent comprising 1-70% by mass of component (a), 30-98.999% by mass of component (b) and 0.001-5% by mass of component (c2).

2. The method according to claim 1, wherein the total amount of component (a), component (b) and component (c2) is 95-100% by mass.

3. The method according to claim 1 or 2, which further comprises, as component (d), an organic compound other than

component (a).

4. The method according to claim 3, wherein the amount of component (d) is 5% or less by mass.

5. The method according to claim 3 or 4, wherein component (d) is a perfume component (d1).

6. The method according to claim 5, wherein component (d1) is at least one compound selected from (d1-1) jasmonoids, chained sesquiterpene alcohols and chained diterpene alcohols.

7. The method according to anyone of claims 1 to 6, which is able to remove at least one selected from mites, chironomids, cockroaches, dust and dirt resulting from dead bodies and dung thereof, fragments of body hairs of pets, pollen and spores of fungi.

8. The method according to anyone of claims 1 to 7, comprising spraying the allergen-removing agent and bringing it into contact with an object to be treated which an allergen adheres to.

9. The method according to anyone of claims 1 to 8, comprising spraying the allergen-removing agent with a trigger type sprayer and bringing it into contact with an object to be treated which an allergen adheres to.

10. The method according to anyone of claims 1 to 9, comprising wiping off the allergen-removing agent to remove allergen from the object to be treated, together with solid precipitates produced by drying the contacted place.

11. The method according to anyone of claims 1 to 10, comprising wiping off the allergen-removing agent with a cleaner to remove allergen from the object to be treated, together with solid precipitates produced by drying the contacted place.

12. The method according to anyone of claims 1 to 11, wherein component (c2) is at least one selected from sodium sulfate and magnesium sulfate.

## Patentansprüche

1. Verfahren zur Entfernung von Allergenen mit einem Allergen-entfernenden Mittel, das das in Kontakt bringen einer flüssigen Zusammensetzung, die durch Entfernen ihrer flüssigen Komponenten einen Feststoff erzeugt, mit einem zu behandelnden Gegenstand, in dem ein Allergen vorhanden ist, und das Trocknen der kontaktierten Stelle zur Bildung eines ausgefällten Feststoffs und das Entfernen des Allergens von dem Gegenstand zusammen mit dem ausgefällten Feststoff umfasst,
wobei das Allergen-entfernende Mittel die folgende Komponente (a), Komponente (b) und Komponente (c2) umfasst: worin
Komponente (a) mindestens eine Verbindung ist, die aus der Gruppe ausgewählt wird, die aus Ethanol, 1-Propanol und 2-Propanol besteht,
Komponente (b): Wasser und
Komponente (c2): eine Feststoffquellensubstanz, die in mindestens einer der Komponente (a) und der Komponente (b) löslich ist und einen Feststoff durch Verdampfung der flüssigen Komponenten des Allergen-entfernenden Mittels erzeugt,
und die eine Kombination aus einem anorganischen Kation mit einem anorganischen Anion ist, wobei das anorganische Kation mindestens eines ist, das aus der Gruppe aus Alkalimetallionen und Erdalkalimetallionen ausgewählt wird, das anorganische Anion mindestens eines ist, das aus der Gruppe aus einem Sulfation, einem Carbonation und einem Phosphation ausgewählt wird,
wobei das Allergen-entfernende Mittel 1 bis 70 Massen% der Komponente (a), 30 bis 98,999 Massen% der Komponente (b) und 0,001 bis 5 Massen% der Komponente (c2) umfasst.

2. Verfahren gemäß Anspruch 1, worin die Gesamtmenge aus Komponente (a), Komponente (b) und Komponente (c2) 95 bis 100 Massen% beträgt.

3. Verfahren gemäß Anspruch 1 oder 2, das ferner eine von der Komponente (a) verschiedene organische Verbindung als Komponente (d) umfasst.

4. Verfahren gemäß Anspruch 3, worin die Menge der Komponente (d) 5 Massen% oder weniger beträgt.

5. Verfahren gemäß Anspruch 3 oder 4, worin die Komponente (d) eine Parfümkomponente (d1) ist.

6. Verfahren gemäß Anspruch 5, worin die Komponente (d1) mindestens eine Verbindung ist, die aus (d1-1) Jasmoniden, kettenförmige Sesquiterpen-Alkoholen und kettenförmige Diterpen-Alkoholen ausgewählt wird.

7. Verfahren gemäß mindestens einem der Ansprüche 1 bis 6, das zur Entfernung von mindestens einem Vertreter, ausgewählt aus Milben, Zuckmücken, Kakerlaken, Staub und Schmutz, der von toten Körpern und Kot davon herrührt, Fragmenten von Körperhaaren von Haustieren, Pollen und Pilzsporen, geeignet ist.

8. Verfahren gemäß mindestens einem der Ansprüche 1 bis 7, umfassend das Versprühen des Allergen-entfernenden Mittels und das in Kontakt bringen davon mit einem zu behandelnden Gegenstand, an dem ein Allergen haftet.

9. Verfahren gemäß mindestens einem der Ansprüche 1 bis 8, umfassend das Versprühen des Allergen-entfernenden Mittels mit einem Zerstäuber mit Auslöser und das in Kontakt bringen davon mit einem Gegenstand, an dem ein Allergen haftet.

10. Verfahren gemäß mindestens einem der Ansprüche 1 bis 9, umfassend das Abwischen des Allergen-entfernenden Mittels zur Entfernung des Allergens von dem zu behandelnden Gegenstand zusammen mit den festen Niederschlägen, die durch das Trocknen der kontaktierten Stelle erzeugt wurden.

11. Verfahren gemäß mindestens einem der Ansprüche 1 bis 10, umfassend das Abwischen des Allergen-entfernenden Mittels mit einem Reiniger zur Entfernung des Allergens von dem zu behandelnden Gegenstand zusammen mit den festen Niederschlägen, die durch das Trocknen der kontaktierten Stelle erzeugt wurden.

12. Verfahren gemäß mindestens einem der Ansprüche 1 bis 11, worin die Komponente (c2) mindestens ein Vertreter ist, der aus Natriumsulfat und Magnesiumsulfat ausgewählt wird.

## Revendications

1. Procédé d'élimination d'allergènes, avec un agent d'élimination d'allergènes, qui comprend la mise au contact d'une composition liquide qui génère un solide par l'élimination des composants liquides de celle-ci avec un objet à traiter dans lequel un allergène est présent et le séchage de l'endroit mis au contact pour former un solide précipité et l'élimination, de l'objet, de l'allergène conjointement au solide précipité,
l'agent d'élimination d'allergènes comprenant le composant (a), le composant (b) et le composant (c2) suivants : dans lequel
le composant (a) au moins un composé choisi dans le groupe constitué de l'éthanol, du 1-propanol et du 2-propanol,
le composant (b) : l'eau et
le composant (c2) : une substance source de solide qui est soluble dans au moins l'un du composant (a) et du composant (b) et génère un solide par évaporation des composants liquides de l'agent d'élimination d'allergènes, qui est une combinaison d'un cation inorganique avec un anion inorganique, le cation inorganique étant au moins un choisi dans le groupe des ions de métal alcalin, et des ions de métal alcalino-terreux, l'anion inorganique étant au moins un choisi dans le groupe d'un ion sulfate, d'un ion carbonate et d'un ion phosphate,
l'agent d'élimination d'allergènes comprenant 1 à 70 % en masse de composant (a), 30 à 98,999 % en masse de composant (b) et 0,001 à 5 % en masse de composant (c2).

2. Procédé selon la revendication 1, dans lequel la quantité totale de composant (a), de composant (b) et de composant (c2) est de 95 à 100 % en masse.

3. Procédé selon la revendication 1 ou 2, qui comprend en outre, comme composant (d), un composé organique autre que le composant (a).

4. Procédé selon la revendication 3, dans lequel la quantité de composant (d) est de 5 % ou moins en masse.

5. Procédé selon la revendication 3 ou 4, dans lequel le composant (d) est un composant de parfum (d1).

6. Procédé selon la revendication 5, dans lequel le composant (d1) est au moins un composé choisi parmi les jasmonoïdes, les alcools à chaîne sesquiterpène et les alcools à chaîne diterpène (d1-1).

7. Procédé selon l'une quelconque des revendications 1 à 6, qui est capable d'éliminer au moins un choisi parmi les mites, les chironomes, les cafards, la poussière et la saleté résultant des cadavres et des crottes de ceux-ci, les fragments de poils d'animaux domestiques, le pollen et les spores de champignons.

8. Procédé selon l'une quelconque des revendications 1 à 7, comprenant la pulvérisation de l'agent d'élimination d'allergènes et sa mise au contact d'un objet à traiter auquel adhère un allergène.

9. Procédé selon l'une quelconque des revendications 1 à 8, comprenant la pulvérisation de l'agent d'élimination d'allergènes avec un pulvérisateur de type gâchette et sa mise au contact d'un objet à traiter auquel adhère un allergène.

10. Procédé selon l'une quelconque des revendications 1 à 9, comprenant l'essuyage de l'agent d'élimination d'allergènes pour éliminer l'allergène de l'objet à traiter, conjointement aux précipités solides produits par le séchage de l'endroit mis au contact.

11. Procédé selon l'une quelconque des revendications 1 à 10, comprenant l'essuyage de l'agent d'élimination d'allergènes avec un produit de nettoyage pour éliminer l'allergène de l'objet à traiter, conjointement aux précipités solides produits par le séchage de l'endroit mis au contact.

12. Procédé selon l'une quelconque des revendications 1 à 11, dans lequel le composant (c2) est au moins un choisi parmi le sulfate de sodium et le sulfate de magnésium.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 9157116 A
- JP 6279273 A
- JP 61044821 A
- JP 2000063207 A
- JP 53130704 A
- GB 1343312 A
- JP 462934 A
- JP 2001094696 A


### Non-patent literature cited in the description


- Surfactant Handbook. Sangyo Tosho Co., Ltd, 1960, 319
- Kagaku Binran Kiso-Hen. Maruzen Co., Ltd, vol. II
- **Ryoichi Akaboshi.** Chemistry of Perfumes. 16 September 1983
- **Genichi Fuji.** Chemistry and Commodity-Knowledge of Synthetic Perfumes. Kagaku Kogyo Nippoh Co, 06 March 1996
- **Motoki Nakajima.** Practical Knowledge of Perfumes and Perfume-Preparation. Sungyo Tosyo Co., Ltd, 21 June 1995